## Europäisches Patentamt

## European Patent Office

## Office européen des brevets

(19)

(11) Veröffentlichungsnummer: **0 186 751**

**B1**

# (12) EUROPÄISCHE PATENTSCHRIFT

(45) Veröffentlichungstag der Patentschrift:
**06.04.88**

(21) Anmeldenummer: **85113775.2**

(22) Anmeldetag: **29.10.85**

(51) Int. Cl.⁴: **C 12 Q 1/26,** C 12 Q 1/66 //
G01N33/53

(54) **Verfahren zur Initiierung der Lichtemission von Phthalhydrazinen.**

(30) Priorität: **31.10.84 DE 3439742**

(43) Veröffentlichungstag der Anmeldung:
**09.07.86 Patentblatt 86/28**

(45) Bekanntmachung des Hinweises auf die Patenterteilung:
**06.04.88 Patentblatt 88/14**

(84) Benannte Vertragsstaaten:
**AT BE CH DE FR GB IT LI LU NL SE**

(56) Entgegenhaltungen:
EP-A-0 116 454
WO-A-83/03104

CLINICAL CHEMISTRY, Band 31, Nr. 8, August 1985,
Seiten 1335-1341, Winston-Salem, North Carolina,
US; G.H.G. THORPE et al.: "Phenols as enhancers
of the chemiluminescent horseradish peroxidase-
luminol-hydrogen peroxide reaction: application in
luminescence-monitored enzyme immunoassays"

(73) Patentinhaber: **Henning Berlin GmbH Chemie und
Pharmawerk, Komturstrasse 19- 20, D-1000 Berlin
42 (DE)**

(72) Erfinder: **Hantke, Uwe, Dipl.- Chem.,
Elswigstrasse 9 A, D-2400 Lübeck 1 (DE)**
Erfinder: **Wood, William Graham, Dr.,
Lerchenredder 1, D-2401 Gross Grönau (DE)**

(74) Vertreter: **Werner, Hans- Karsten, Dr.,
Deichmannhaus am Hauptbahnhof, D-5000 Köln 1
(DE)**

**Beschreibung**

Gegenstand der vorliegenden Erfindung ist ein Verfahren zur Initiierung der Lichtemission durch Oxidation von Phthalhydrazinen in Lumineszenz-Immuno-Tests durch Zugabe von Pseudoperoxidase, wässrigem Natriumhydroxid und wässrigem Peroxid.

Diese Initiierung durch die drei Lösungen ist bereits ausführlich beschrieben worden in J. Clin. Chem. Clin. Biochem. 21, S. 789-797 (1983), Loc.cit. 22, S. 337-347 (1984) und Loc.cit. 22, S.349-356 (1984). Phthalhydrazine wie das 6[N-(4-Aminobutyl-N-ethyl)isoluminol]he-misuccianmid (ABEI - H) haben bei Lumineszenz-Immuno-Tests eine Reihe von Vorteilen, jedoch ist es bislang notwendig, die Initiierung durch getrennte Zugabe von drei Reagenzlösungen vorzunehmen, da die Stabilität, Durchführbarkeit und Reproduzierbarkeit des Lumineszenz-Immuno-Tests von dem gleichbleibenden Eigenschaften der drei Reagenzlösungen abhängig ist.

Andere Lumineszenzreaktionen, wie sie für Acridiniumester und Fluoreszeinisothiocyanat beschrieben wurden, können zwar durch nur eine Lösung initiiert werden, jedoch zeigen diese Systeme bei der Anwendung in Immuno-Tests zahlreiche Nachteile.

Versuche, die drei die Lumineszenz von beispielsweise ABEI-H auslösenden Reagenzien, nämlich Pseudoperoxidase, Natronlauge und Wasserstoffperoxid zu einer Lösung zu vereinen, haben gezeigt, daß sich eine derartige Mischung so rasch zersetzt, daß sie unbrauchbar wird und daher zu keinen reproduzierbaren Ergebnissen führt. Auch Versuche, die nur die Natronlauge und das Peroxid zu einer Lösung von alkalischem Wasserstoffperoxid vereinen, haben bei Phthalhydrazinen zunächst zu keinen brauchbaren Ergebnissen geführt.

Überraschenderweise wurde jetzt gefunden, daß sich eine Mischung aus Natriumhydroxid und Wasserstoffperoxid oder anderen Peroxiden nach etwa 20 Minuten stabilisiert und danach bis zu 10 Stunden brauchbar bleibt. Die Lumineszenz von Phthalhydrazinen wie ABEI-H steigt während dieser ersten 20 Minuten an und bleibt danach konstant. Diese Signalverstärkung führt in Lumineszenz-Immuno-Tests mit Phthalhydrazinen somit zu einer wesentlichen Verbesserung der Empfindlichkeit und unteren Nachweisgrenzen. Vorzugsweise wird daher das Gemisch aus Natriumhydroxid und Peroxid verwendet, wenn es 0,5 bis 8 Stunden alt ist.

Weiterhin hat sich gezeigt, daß die Pseudoperoxidase ohne weiteres einem Testansatz auch vor der Messung im Luminometer zugegeben werden kann, so daß die Initiierung durch Zugabe von nur einer Lösung, nämlich einer 0,3 bis 10 Stunden alten Lösung von alkalischem Peroxid direkt in der Meßposition des Luminometers möglich ist. Überraschenderweise wurde hierbei weiterhin festgestellt, daß bei dem erfindungsgemäßen Verfahren die Lichtemission gegenüber dem Originalsystem, bei dem die drei initiierenden Reagenzien nacheinander zugegeben werden, 2- bis 5-fach höher ist. Die höhere Lichtemission wird dabei erzielt, ohne den Reagenzienleerwert in störender Weise zu erhöhen.

Gegenstand der vorliegenden Erfindung ist somit ein Verfahren zu Initiierung der Lichtemissionen durch Oxidation von Phthalhydrazinen in Lumineszenz-Immuno-Tests durch Zugabe von Pseudoperoxidase, wässrigem Natriumhydroxid und wässrigem Peroxid, dadurch gekennzeichnet, daß die Pseudoperoxidase vor der Messung dem Testansatz zugegeben wird und die Initiierung der Lichtemission durch Zugabe einer 0,3 bis 10 Stunden alten Lösung von alkalischem Peroxid erfolgt. Vorzugsweise wird das Verfahren durchgeführt mit einer alkalischen Peroxidlösung, die ca. 0,33 Mol Natriumhydroxid und ca. 0,1 Gew.-% Wasserstoffperoxid enthält. Das Peroxid kann in besonders einfacher Weise durch Auflösung von Perhydrittabletten (Harnstoff-Peroxid) in Wasser gewonnen werden.

Durch die Möglichkeit, die Initiierung mit nur einer Lösung durchzuführen, kann das Lumineszenzsystem auf Basis von Phthalhydrazinen auch auf geschlossene, automatisierte Systeme übertragen werden. Hierdurch läßt sich der Anwendungsbereich des Systems für die Praxis erheblich erweitern. Durch die an sich hohe Genauigkeit und Empfindlichkeit und die erfindungsgemäß beobachtete höhere Lichtemission wird das erfindungsgemäße Verfahren für die Praxis besonders wertvoll. Die Lebensdauer der alkalischen Wasserstoffperoxidlösung von 0,3 bis 10 Stunden reicht für die Anforderungen der Praxis völlig aus. Sofern die alkalische Peroxidlösung noch zu frisch ist, erzielt man jedoch stark verfälschte und unbrauchbare Ergebnisse. Eine Lebensdauer von 8 bis 10 Stunden reicht völlig aus, den Tagesbedarf eines Routine-Labors auf einmal anzusetzen und erfindungsgemäß zu verwenden.

Ein weiterer Vorteil des erfindungsgemäßen Verfahrens besteht darin, daß nunmehr auch für dieses Lumineszenzsystem Geräte verwendet werden können mit nur einer Injektions-Einrichtung (Dispenser). Derartige Geräte sind billiger in der Anschaffung, einfacher in der Handhabung und weniger störanfällig, womit eine weitere Kostensenkung und Qualitätssteigerung der Analytik mit diesem Lumineszenzsystem möglich ist.

Das erfindungsgemäße Verfahren ist in den nachfolgenden Beispielen näher erläutert, wobei durch Vergleichsversuche mit der bekannten Methode, d.h. durch getrennte Zugabe von drei Reagenz-Lösungen die überraschenden Vorteile herausgestellt sind.

**Beispiel 1**

Verwendet wurden Pseudoperoxidase (MP 11) der Firma Sigma, München. Als Peroxidquelle wurden in Wasser gelöste Tabletten (Perhydrittabletten der Firma Merck, Darmstadt) verwendet. Das Ferritin-Antiserum stammte von der Firma Dakopotts, Hamburg und Proma (Seward Antibodies) Augsburg wie bereits in J.Clin. Chem. Clin. Biochem. 22, S. 349-356 (1984) publiziert. Auch die Durchführung des Lumineszenz-Immuno-Tests für die Bestimmung von Ferritin kann dieser Publikation entnommen werden. Die Messungen erfolgten in einem Luminometer der Firma Berthold LB-950.

Folgende Lösungen und Injektionsvolumina wurden für die Injektion der drei einzelnen Lösungen in der bekannten 3-Dispenserversion verwendet:

Dispenser 1:

100 µl NaOH - 1 Mol/l (Lösung 1).

Dispenser 2:

120 µl Pseudoperoxidase (MP 11) - 5 µMol/l in Phosphatpuffer (0,05 Mol/l) enthaltend Rinderserumalbumin (0,4 g/l), Natriumchlorid (0,1 Mol/l) und Natriumazid (15 mMol/l). Der pH-Wert betrug 8,0 (Lösung 2).

Im Dispenser 3:

300 µl Wasserstoffperoxid - 1 Perhydrittablette wurde in 200 ml bidestilliertem Wasser gelöst und ergab eine etwa 1,5 ml/l Lösung von $H_2O_2$ in Wasser (Lösung 3). Das Volumen der in den Testansätzen (Röhrchen) vorgelegten NaCl-Lösung (0,15 Mol/l) zur Überschichtung der Polystyrolkugeln betrug 300 µl.

Bei der erfindungsgemäßen Initiierung mit nur einer Lösung wurde den Testansätzen mit dem Dispenser 3 350 µl einer Mischung bestehend aus Natriumhydroxid (1 Volumenteil der Lösung 1) und Peroxid (2 Volumenteile der Lösung 3) zugesetzt.

Die in den Testansätzen (Röhrchen) vorgelegte NaCl-Lösung (0,15 Mol/l) zur Überschichtung der Polystyrolkugeln bestand aus 300 µl einer Mischung der Pseudoperoxidase (1 Volumenteil der Lösung 2) und Natriumchlorid (2 Volumenteile) der oben angegebenen Konzentrationen.

Die Bezeichnung Dispenser 3 wird benutzt für den Dispenser, der in die die Meßkammer injiziert. Dispenser 2 injiziert eine Position vor der Kammer und Dispenser 1 injiziert 14 Positionen vor der Meßkammer.

Die Meßergebnisse sind in der nachfolgenden Tabelle 1 zusammengestellt, wobei die Werte unter 3 D nach dem Stand der Technik mit 3 Dispensern und unter 1 D erfindungsgemäß mit nur einem Dispenser ermittelten Lumineszenz-Signale repräsentieren.

**Tabelle 1**

| Standardkurve Ferritin - µg/l | Beginn der Bestimmung | | Ende der Bestimmung | |
|---|---|---|---|---|
| | 3 D | 1 D | 3 D | 1 D |
| 0 | 119/130 | 205/205 | 124/129 | 209/223 |
| 5 | 142/150 | 237/255 | 145/146 | 258/271 |
| 20 | 322/308 | 415/471 | 347/358 | 455/449 |
| 50 | 742/717 | 1360/1417 | 710/693 | 1413/1387 |
| 200 | 2410/2495 | 3827/4113 | 2288/2406 | 3824/3880 |
| 500 | 4795/4952 | 7722/8157 | 4816/5007 | 7658/7229 |
| 1000 | 6836/6693 | 12339/11971 | 6728/6357 | 12000/12152 |

Alle Lumineszenz-Signale sind in Berthold Lichteinheiten x $10^{-3}$ angegeben. Die Signale am Ende der Bestimmung wurden ca. 2,5 Stunden nach dem Start (Beginn der Bestimmung) gemessen.

Die Überprüfung der Werte durch Kontrollseren ergab folgende Werte:

**Tabelle 2**

| | Beginn der Bestimmung | | | Ende der Bestimmung | |
|---|---|---|---|---|---|
| | 3 D | 1 D | | 3 D | 1 D |
| Serum A | 13,7 | 14,3 | 13,4 | 12,4 | |
| Serum B | 82,8 | 79,8 | 77,4 | 78,8 | |
| Serum C | 18,3 | 21,7 | 22,2 | 25,8 | |
| Serum D | 45,6 | 49,5 | 47,3 | 50,5 | |
| Serum E | 119 | 126 | 124 | 133 | |

Alle Werte sind in µg/l angegeben (Mittelwerte von Doppelbestimmungen ermittelt aus je 5 Testansätzen).

Eine Untersuchung der Methode mit verschieden lange gealterten alkalischen Peroxidlösungen zeigte, daß diese erst nach etwa 20 Minuten nahezu konstante Werte zeigten, die sich auch nach 6 bis 8 Stunden kaum änderten. Lösungen die älter als 18 Stunden waren, waren nicht mehr zu verwenden. Das Gemisch aus Natriumchlorid und Pseudoperoxidase war bis zu 96 Stunden haltbar.

**Patentansprüche**

1. Verfahren zur Initiierung der Lichtemission durch Oxidation von Phthalhydrazinen in Lumineszenz-Immuno-Tests durch Zugabe von Pseudoperoxidase, wässrigem Natriumhydroxid und wässrigem Peroxid, dadurch gekennzeichnet, daß die Pseudoperoxidase vor der Messung dem Testansatz zugegeben wird und die Initiierung der Lichtemission durch Zugabe einer 0,3 bis 10 Stunden alten Lösung von alkalischem Peroxid erfolgt.

2. Verfahren gemäß Anspruch 1, dadurch gekennzeichnet, daß die alkalische Peroxidlösung ca. 0,33 Mol Natriumhydroxid und ca. 0,1 Gew.-% Wasserstoffperoxid enthält.

3. Verfahren gemäß Anspruch 1 oder 2, dadurch gekennzeichnet, daß die Zugabe der Lösung in der Meßposition eines Luminometers erfolgt.

4. Verwendung einer 0,3 bis 10 Stunden alten alkalischen Peroxidlösung enthaltend ca. 0,33 Mol Natriumhydroxid und ca. 0,1 Gew.-% Wasserstoffperoxid zur Initiierung der Lichtemission durch Oxidation von Phthalhydrazinen in Lumineszenz-Immuno-Tests.

**Claims**

1. A process for initiating the light emission by oxidation of phthalohydrazines in luminescence immuno-tests by means of the addition of pseudoperoxidase, aqueous sodium hydroxide and aqueous peroxide, characterized in that the pseudoperoxidase is added to the test batch prior to the measurement and the initiation of the light emission is effected by adding a solution which is from 0.3 to 10 hours old of alkaline peroxide.

2. The process according to claim 1, characterized in that the alkaline peroxide solution contains about 0.33 moles of sodium hydroxide and about 0.1% by weight of hydrogen peroxide.

3. The process according to claims 1 and 2, characterized in that the addition is effected in the measuring position of a luminometer.

4. Use of an alkaline peroxide solution which is from 0.3 to 10 hours old and contains about 0.33 moles of sodium hydroxide and about 0.1% by weight of hydrogen peroxide for initiating the light emission by oxidation of phthalohydrazines in luminescence immuno-tests.

**Revendications**

1. Procédé d'initiation des émissions de lumière par oxydation de phtalohydrazines dans des tests d'immunoluminescence en ajoutant de la pseudoperoxydase, de l'hydroxyde de sodium en solution aqueuse et du péroxyde en solution aqueuse, caractérisé en ce que la pseudoperoxydase est ajoutée à la préparation de test avant la mesure et en ce que l'initiation de l'émission de lumière est obtenue par addition d'une solution de peroxyde alcaline vieille de 0,3 à 10 heures.

2. Procédé selon la revendication 1, caractérisé en ce que la solution de peroxyde alcaline contient environ 0,33 mole d'hydroxyde de sodium et environ 0,1 % en poids de peroxyde d'hydrogène.

3. Procédé selon la revendication 1 ou 2, caractérisé en ce que l'addition de la solution s'effectue dans la position de mesure d'un luminomètre.

4. Emploi d'une solution de peroxyde alcaline, vieille de 0,3 à 10 heures, qui contient environ 0,33 moles d'hydroxyde de sodium et environ 0,1 % en poids de peroxyde d'hydrogène pour initiation de l'émission de lumière par oxydation de phtalohydrazines dans des tests d'immunoluminescence.